# EUROPÄISCHE PATENTSCHRIFT

(11) **EP 0 676 389 B1**
(45) Veröffentlichungstag und Bekanntmachung des Hinweises auf die Patenterteilung: **01.09.1999**
(21) Anmeldenummer: 95104926.1
(22) Anmeldetag: 03.04.1995
(51) Int. Cl.: C07C 51/58, C07C 57/76

(54) **Verfahren zur Herstellung von 0-Chloromethylbenzoesäurechloriden**
Process for preparing O-chloromethyl benzoic acid chloride
Procédé pour la préparation du chlorure de l'acide O-chlorométhyl benzoique

(30) Priorität: 11.04.1994 DE 4412316
(43) Veröffentlichungstag der Anmeldung: 11.10.1995
(73) Patentinhaber: BASF AKTIENGESELLSCHAFT, 67056 Ludwigshafen (DE)
(72) Erfinder: Isak, Heinz, Dr., D-67459 Böhl-Iggelheim (DE); Bayer, Herbert, Dr., D-68159 Mannheim (DE); Keil, Michael, Dr., D-67251 Freinsheim (DE); Wettling, Thomas, Dr., D-67117 Limburgerhof (DE)

(56) Entgegenhaltungen:
- EP-A- 0 583 589
- DE-A- 4 311 722
- DATABASE WPI Week 9309 Derwent Publications Ltd., London, GB; AN 93-071055 & JP-A-05 017 391 (SHOWA DENKO KK) , 26.Januar 1993
- FIESER AND FIESER'S 'Reagents for Organic Synthesis' , J. WILEY & SONS , NEW YORK * Seite 297 : Thionylchloride *

## Beschreibung

Die vorliegende Erfindung betrifft ein Verfahren zur Herstellung von o-Chlormechylbenzoesaurechloriden der Formel I 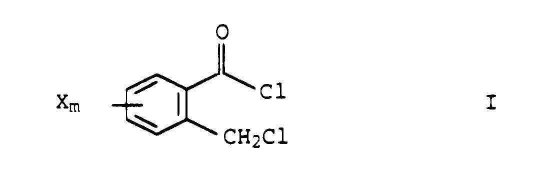 in der
- m: 0 oder eine ganze Zahl von 1-4 bedeutet und
- X: für Halogen oder C-organische Reste steht,
durch Umsetzung eines entsprechendes Lactons der Formel II

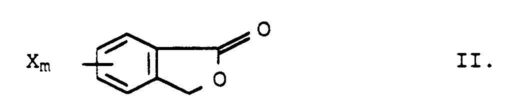

Aus der Literatur ist die Umsetzung von aliphatischen Lactonen mit Phosgen in Gegenwart von Pyridin (US-A 2,778,852), quartären Ammoniumsalzen (DE-A 36 24 258) oder Phosphinoxyden (DE-A 39 27 146) als Katalysator bekannt. Außerdem wird die Umsetzung von Phthalid zu o-Chlormethylbenzoesäurechlorid mittels Phosphorpentachlorid in Gegenwart von Zink-II-chlorid beschrieben [CA 106: 84,307y (1987)].

Sowohl Phosgen als auch Phosphorpentachlorid sind im Hinblick aus eine großtechnische Herstellung entsprechender Carbonsäurechloride wegen ihrer Giftigkeit oder der Giftigkeit der daraus entstehenden Abbauprodukte bedenklich. Die Phosgenierung, wie z.B. in EP-A 583 589 beschrieben, erfolgt bei 170°C. Dies bedeutet, man benötigt ein sehr kostenintensive Kühlung bis auf -70°C, um aus dem Abgas Phosgen auszukondensieren. Darüber hinaus sind die Sicherheitsauflagen für eine solche Prodinktion so hoch, daß eine wirtschaftliche Neuinvestition nicht möglich ist. Des weiteren zeigt die Praxis bei Phosgenierungen, daß in den technischen Anlagen ein Überschuß an Phosgen zu verwenden ist, der in der Regel deutlich über dem Überschuß liegt, der im Labormaßstab zu verwenden ist. Auch ein Transport von Produkten aus der Phosgenierung unterliegt strengen Sicherheitsanforderungen in bezug auf die Phosgengrenzwerte, so daß oftmals eine aufwendige Austreibung von Restphosgenmengen mittels Stickstoff nötig ist.

Ein Einsatz von Phosphorpentachlorid im industriellen Maßstab scheitert in der Regel an den erheblichen Abwasserproblemen durch die Bildung von phosphorhaltigen Abfallprodukten, z.B. Phosphorsäure und Phosphorsäureester, die das Abwasser belasten.

Der vorliegenden Erfindung lag daher eine Möglichkeit zur Herstellung von Halogencarbonsäurechloriden als Aufgabe zugrunde, welches die vorstehenden Nachteile nicht aufweist und großtechnisch anwendbar ist.

Demgemäß wurde gefunden, daß man o-Chlormethylbenzoesäurechloride der Formel I durch Umsetzung der entsprechenden Lactone der Formel II mit Thionylchlorid in Gegenwart eines Katalysators bei 80-240°C erhält.

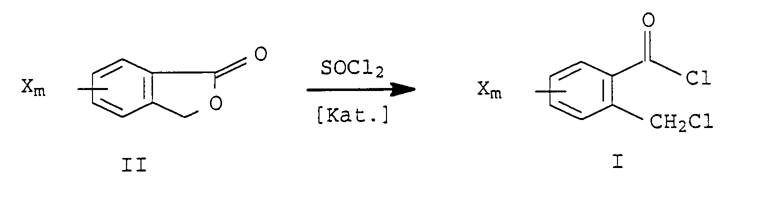

Die Umsetzung benötigt im allgemeinen Mindesttemperaturen von 80°C, wobei die Umsetzungsgeschwindigkeit mit steigender Temperatur steigt. Oberhalb von 300°C setzt verstärkt Zersetzung ein, wodurch oberhalb von 350°C die Ausbeute an Produkt merklich vermindert wird.

Die Umsetzung wird daher üblicherweise bei Temperaturen von 80°C bis 240°C, vorzugsweise 130°C bis 200°C, insbesondere 160°C bis 190°C durchgeführt.

Als Katalysatoren eignen sich besonders organische Stickstoffverbindungen, z.B. stickstoffhaltige aromatische oder aliphatische Heterocyclen wie Pyrazol, Imidazol, Pyridin, Pyrimidin, Pyrazin, Indol, Chinolin, Piperidin, Piperazin und Morpholin, beziehungsweise entsprechende alkylsubstituierte Heterocyclen (z.B. 1-Methylimidazol, Methyl- oder Dimethylpyridine, 1-Decylimidazol, N-Methylpiperidin, N,N'-Dimethylpiperazin, N-Methylmorpholin), tertiäre aliphatische oder aromatische Amine, z.B. Tri-(C₁-C₆-alkyl)amine (Trimethylamin, Triethylamin, Tripropylamin, Tri-(1-methylethyl)-amin, Tributylamin, Tri-(1-methylpropyl)-amin, Tri-(2-methylpropyl)-amin, N,N-Dimethylanilin oder Amine, welche zwei oder drei ungleiche Alkylreste mit 1 bis 6 C-Atomen oder zwei gleiche oder ungleiche Alkylreste mit 1 bis 6 C-Atomen oder zwei gleiche oder ungleiche Alkylreste mit 1 bis 6 C-Atomen und einen Arylrest, z.B. Phenyl- oder Benzylrest, tragen), sowie entsprechende quartäre Ammoniumsalze, insbesondere quartäre Ammoniumchloride (z.B. Tetramethylammoniumchlorid, Tetrabutylammoniumchlorid, Benzyltrimethylammoniumchlorid), N,N'-Tetra-(C₁-C₆-alkyl/aryl)-harnstoffe oder - guanidine (z.B. N,N,N',N'-Tetramethylharnstoff, N,N,N',N'-Tetrabutylharnstoff, N,N'-dimethyl-N,N'-diphenylharnstoff, Tetramethylginanidin, Tetraphenylguanidin) sowie insbesondere N,N-Di-(C₁-C₆-alkyl/aryl)-form-amide (z.B. Dimethylformamid, Diethylformamid, Dipropylformamid, Di-(1-Methylethyl)-formamid, Dibutylformamid, Di-(1-Methylpropyl)-formamid, Di-(2-Methylpropyl)-formamid, Methylphenylformamid).

Der Katalysator sollte Mengen von 0,1 mol-% bezogen auf das eingesetzte Lacton nicht unterschreiten. Die Umsetzungsgeschwindigkeit steigt im allgemeinen mit steigender Menge an Katalysator. Ab Mengen von ca. 25 mol-% ist jedoch in der Regel keine merkliche verbesserung zu erreichen.

Der Katalysator wird daher üblicherweise in Mengen von 0,1 bis 25 mol-%, vorzugsweise 0,15 bis 10 mol-%, insbesondere 0,5 bis 5 mol-%, bezogen auf die eingesetzte Mengen an Lacton II, zugesetzt.

Das Thionylchlorid kann gleichzeitig als Losungsmittel für das Lacton dienen und wird in einem solchen Fall in einem entsprechenden Überschuß verwendet, wobei sich die Höhe des Überschusses nach dem Lösungsverhalten des Lactons richtet. Üblicherweise braucht ein Überschuß von 10 mol an Thionylchlorid pro mol Lacton II nicht überschritten zu werden. Sofern die Umsetzung in einem inerten Lösungsmittel durchgeführt wird, werden geringere Mengen an Thionylchlorid verwendet.

Üblicherweise verwendet man das Thionylchlorid in Mengen von 0,8 bis 10 mol, vorzugsweise 0,8 bis 5 mol, insbesondere 1 bis 2 mol, pro mol Lacton II.

Die Reaktion kann unter Normaldruck oder unter Druck durchgeführt werden (vorzugsweise 0,01 bis bis 50 bar, insbesondere 0,1 bis 5 bar Überdruck).

Bei der Umsetzung unter Normaldruck hat es sich als besonders vorteilhaft erwiesen, die Umsetzung in Gegenwart von Chlorwasserstoff oder einer aus Thionylchlorid Chlorwasserstoff freisetzenden Verbindung durchzuführen. Durch diesen Zusatz wird eine erhebliche Beschleunigung der Umsetzung und eine Verbesserung des Umsatzes erreicht. Als Chlorwasserstoff freisetzende Verbindung eignet sich insbesondere Wasser.

Bei der Umsetzung in Gegenwart von Chlorwasserstoff können 5 bis 100 mol-% an Chlorwasserstoff bezogen auf das Lacton II, vorzugsweise 10 bis 50 mol-%, insbesondere 20 bis 40 mol-%, eingesetzt werden. Die Verwendung von größeren Mengen an Chlorwasserstoff ist einerseits aus wirtschaftlichen Gründen unvorteilhaft, andererseits können durch die Verwendung größerer Mengen unerwünschte "Stripp-Effekte" auftreten, d.h. größere Mengen an Thionylchlorid werden beim Sieden mitgerissen und dadurch in den Kühler Verschleppt. Derartige Effekte würden zu Ausbeuteeinbußen führen.

Bei der Verwendung Von Wasser als Chlorwasserstoff freisetzende verbindung finden üblicherweise 0,5 bis 50 Gew.-%, vorzugsweise 0,5 bis 25 Gew.-%, insbesondere 1 bis 25 Gew.-% Verwendung.

Das erfindungsgemäße Verfahren wird üblicherweise so durchgeführt, daß man eine Mischung aus Lacton II und Katalysator, die ggf. ein inertes Lösungsmittel enthalten kann, bei der Umsetzungstemperatur nach und nach mit der erforderlichen Menge an Thionylchlorid versetzt. Dabei abdestillierendes Thionylchlorid kann in die Reaktion zurückgeführt werden.

Bei der zusätzlichen Verwendung von Chlorwasserstoff oder einer Chlorwasserstoff freisetzenden Verbindung erfolgt die Zugabe gleichseitig aber getrennt zum Thionylchlorid.

Wenn Thionylchlorid als Lösungsmittel dient, kann das Lacton II mit Thionylchlorid vorgelegt werden und bei 120 bis 170°C gegebenenfalls mit Wasser oder einer Chlorwasserstoff freisetzenden Verbindung versetzt werden.

Als inerte Lösungsmittel (Verdünnungsmittel) können grundsätzlich alle organischen Lösungsmittel eingesetzt werden, die sich unter den Umsetzungsbedingungen inert verhalten und deren Siedepunkt die erforderliche Umsetzungstemperatur erlaubt. Beispiele hierfür sind hochsiedende Kohlenwasserstoffe wie Cumol, Paraffinöl und Naphthalin oder auch chlorierte Kohlenwasserstoffe wie Dichlorbenzol und Trichlorbenzol.

Nach Abschluß der Umsetzung wird überschüssiges Thionylchlorid und ggf. Lösungsmittel destillativ (ggf. bei vermindertem Druck) entfernt. Die Produkte sind durch fraktionierte Destillation rein erhältlich.

Das erfindungsgemäße Verfahren eignet sich zur Herstellung von o-Chlormethylbenzoesäuren der Formel I 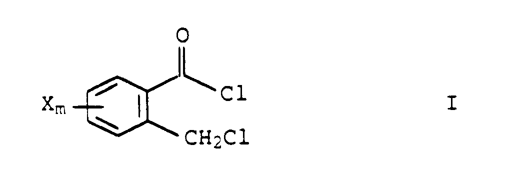 in der
- m: 0 oder eine ganze Zahl von 1-4 bedeutet und
- X: für Halogen oder C-organische Reste steht, wobei die Gruppen X verschieden sein können, wenn der Wert von m größer als 1 ist.

Unter Halogen sind hierbei Fluor, Chlor, Brom und Jod, bevorzugt Fluor, Chlor und Brom, insbesondere Fluor und Chlor zu verstehen. Als C-organische Reste kommen grundsätzlich alle Reste in Betracht, die sich unter den Umsetzungsbedingungen inert verhalten. Beispiele hierfür sind:
- Alkylgruppen, z.B. mit 1 bis 20 Kohlenstoffatomen, bevorzugt geradkettige oder verzweigte Alkylgruppen mit 1 bis 8, insbesondere 1 bis 6 Kohlenstoffatomen;
- Alkenylgruppen, z.B. mit 2 bis 20 Kohlenstoffatomen, bevorzugt geradkettige oder verzweigte Alkenylgruppen mit 2 bis 8, insbesondere 2 bis 6 Kohlenstoffatomen;
- Alkinylgruppen, z.B. mit 2 bis 20 Kohlenstoffatomen, bevorzugt geradkettige oder verzweigte Alkinylgruppen mit 2 bis 8, insbesondere 2 bis 6 Kohlenstoffatomen,
wobei die vorstehend genannten Gruppen ihrerseits partiell oder vollständig halogeniert sein können und/oder weitere unter den Umsetzungsbedingungen stabile Reste tragen können (z.B. Cycloalkyl, Aryl und Heteroaryl).

Weitere Beispiele für C-organische Reste sind:
- Cycloalkylgruppen, z.B. mit 3 bis 10 Kohlenstoffringgliedern, bevorzugt C₃-C₇-Cycloalkylgruppen;
- Arylgruppen, z.B. Phenyl, Naphthyl oder Anthryl,
wobei die vorstehend genannten cyclischen Reste ihrerseits partiell oder vollständig halogeniert sein können und/oder weitere unter den Umsetzungsbedingungen stabile Reste tragen können (z.B. Alkyl, Alkenyl, Alkinyl, Cycloalkyl, Aryl oder Heteroaryl).

Außerdem sind unter C-organischen Resten auch, für den Fall, daß m gleich oder größer als 2 ist,
- Alkylenketten mit 1 bis 6 C-Atomen (vorzugsweise 1, 3 oder 4 C-Atomen) oder
- Alkenylenketten mit 4 C-Atomen (z.B. But-1-en-1,4-diyl, But-2-en- 1,4-diyl oder Buta-1,3-dien-1,4-diyl)
zu verstehen, die an benachbarte Positionen des Phenylrings gebunden sind, wobei diese Reste ihrerseits partiell oder vollständig halogeniert sein können und/oder weitere unter den Umsetzungsbedingungen stabile Reste tragen können (z.B. Alkyl, Alkenyl, Alkinyl, Cycloalkyl, Aryl oder Heteroaryl).

Die nach dem erfindungsgemäßen Verfahren erhältlichen o-Chlormethylbenzoesäurechloride sind wertvolle Zwischenprodukte für die Synthese von Farbstoffen, Arzneimitteln und insbesondere Pflanzenschutzmitteln.

### Verfahrensbeispiele

### Allgemeine Verfahrensvorschrift

In einem Rührreaktor mit Kühlung und einer Zufuhrmöglichkeit für Chlorwasserstoff oder Wasser wurden x g Phthalid und 15 mol-% des Katalysators vorgelegt. Die Mischung wurde auf 160°C bis 180°C (Innentemperatur) erhitzt und bei dieser Temperatur im Laufe von t Stunden mit y g Thionylchlorid und ca. 5 bis 10 l/h Chlorwasserstoff (gasförmig) versetzt. Bei der Umsetzung abdestillierendes Thionylchlorid wurde auskondensiert und in die Reaktion zurückgeführt.

Nach beendeter Reaktion wurde das Reaktionsgemisch einer weitere Stunde bei der Umsetzungstemperatur gehalten, bevor das überschüssige Thionylchlorid abdestilliert wurde. Die reinen Produkte wurden durch fraktionierte Destillation erhalten.

Die Einzelheiten der Versuche sind in der nachfolgenden Tabelle (Beispiele 1 bis 5) zusammengestellt.

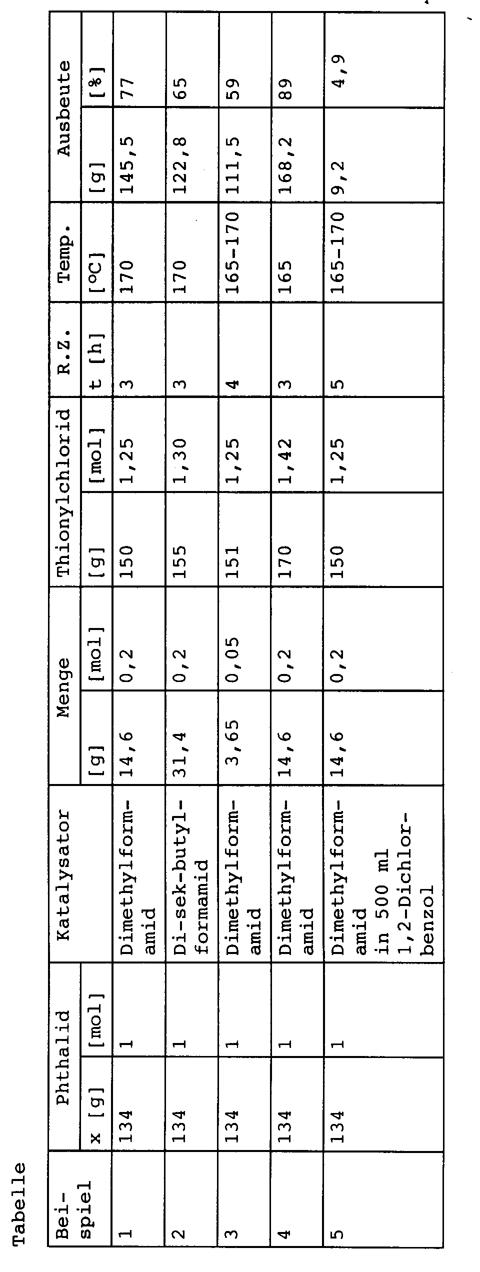

### Beispiel 6

In einem Rührreaktor (Autoklav mit Rührwerk) mit Kühlung und einer Zufuhrmöglichkeit für Wasser oder Chlorwasserstoff wurden 134 g (1 mol) Phthalid und 0,1 mol (10 mol-%) Katalysator (Dimethylformamid) in 119 g (1 mol) Thionylchlorid gelöst und unter Druckhaltung auf 160°C erwärmt. Bei dieser Temperatur wurde 8 Stunden gerührt und dann abgekühlt. Anschließend wurde das Reaktionsgemisch fraktioniert destilliert. Das abgetrennte Thionylchlorid wurde wieder eingesetzt.
Ausbeute: 158 g (83,7 % 2-Chlormethyl-benzoylchlorid)
Sdp.: 120°-125°C (bei 10 mbar)

## Patentansprüche

1. Verfahren zur Herstellung von o-Chlormethylbenzoesäurechloriden der Formel I 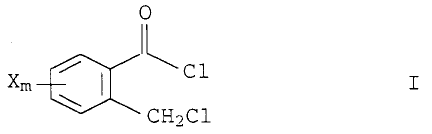 in der
m 0 oder eine ganze Zahl von 1-4 bedeutet und
X für Halogen oder C-organische Reste steht, wobei die Gruppen X verschieden sein können, wenn der Wert von m größer als 1 ist,
dadurch gekennzeichnet, daß man ein entsprechendes Lacton der Formel II 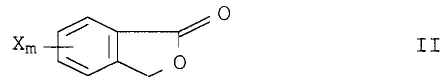 in Gegenwart eines Katalysators bei Temperaturen von 80°C bis 240°C mit Thionylchlorid umsetzt.

2. Verfahren nach Anspruch 1, dadurch gekennzeichnet, daß man als Katalysator organische Stickstoffverbindungen verwendet.

3. Verfahren nach Anspruch 1, dadurch gekennzeichnet, daß man 0,1 bis 25 mol-% eines Katalysators pro mol des Lactons II verwendet.

4. Verfahren nach Anspruch 1, dadurch gekennzeichnet, daß man 0,8 bis 10 mol Thionylchlorid pro mol des Lactons II verwendet.

5. Verfahren nach Anspruch 1, dadurch gekennzeichnet, daß man die Umsetzung in Gegenwart von 5 bis 100 Gew-% Chlorwasserstoff oder einer aus Thionylchlorid Chlorwasserstoff freisetzenden Verbindung bezogen auf das eingesetzte Thionylchlorid durchführt.

6. Verfahren nach Anspruch 1, dadurch gekennzeichnet, daß man die Umsetzung in Gegenwart von 1 bis 25 Gew.-% Wasser bezogen auf das eingesetzte Thionylchlorid durchführt.

## Claims

1. A process for preparing o-chloromethylbenzoyl chlorides of the formula I 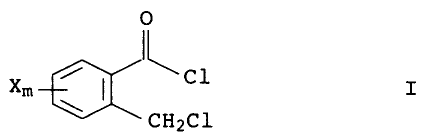 where
m is 0 or an integer from 1 to 4 and
X is halogen or C-organic radicals, it being possible for the groups X to be different when m is greater than 1,
which comprises reacting a corresponding lactone of the formula II 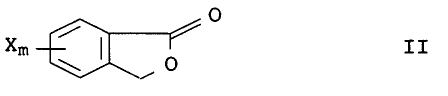 with thionyl chloride at from 80°C to 240°C in the presence of a catalyst.

2. A process as claimed in claim 1, wherein organic nitrogen compounds are used as catalyst.

3. A process as claimed in claim 1, wherein 0.1 - 25 mol% of a catalyst are used, based on the lactone II.

4. A process as claimed in claim 1, wherein 0.8 - 10 mol of thionyl chloride are used per mol of the lactone II.

5. A process as claimed in claim 1, wherein the reaction is carried out in the presence of 5 - 100% by weight of hydrogen chloride, or of a compound which liberates hydrogen chloride from thionyl chloride, based on the thionyl chloride.

6. A process as claimed in claim 1, wherein the reaction is carried out in the presence of 1 - 25% by weight of water based on the thionyl chloride.

## Revendications

1. Procédé pour la préparation de chlorures d'acides o-chlorométhylbenzoïques de formule I 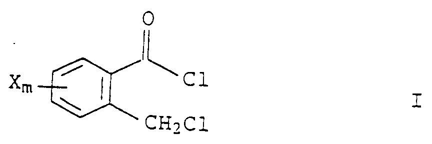 dans laquelle
m est égal à 0 ou représente un nombre entier allant de 1 à 4 et
X représente un halogène ou un radical organique carboné, les substituants X pouvant être différents lorsque m est supérieur à 1,
caractérisé par le fait que l'on fait réagir une lactone correspondante de formule II 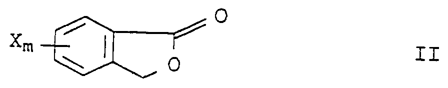 avec le chlorure de thionyle à des températures de 80 à 240°C en présence d'un catalyseur.

2. Procédé selon la revendication 1, caractérisé par le fait que le catalyseur utilisé est un dérivé organique azoté.

3. Procédé selon la revendication 1, caractérisé par le fait que l'on utilise de 0,1 à 25 mol % d'un catalyseur par mot de la lactone II.

4. Procédé selon la revendication 1, caractérisé par le fait que l'on met en oeuvre de 0,8 à 10 mot de chlorure de thionyle par mot de lactone II.

5. Procédé selon la revendication 1, caractérisé par le fait que l'on effectue la réaction en présence de 5 à 100 % en poids de chlorure d'hydrogène ou d'un composé libérant du chlorure d'hydrogène à partir du chlorure de thionyle, par rapport au chlorure de thionyle mis en oeuvre.

6. Procédé selon la revendication 1, caractérisé par le fait que l'on effectue la réaction en présence de 1 à 25 % en poids d'eau par rapport au chlorure de thionyle mis en oeuvre.
